# EUROPEAN PATENT APPLICATION

(11) **EP 0 729 947 A1**
(43) Date of publication of application: **04.09.1996**
(21) Application number: 96810099.0
(22) Date of filing: 19.02.1996
(51) Int. Cl.: C07D 211/74

(54) **Process for the methylation of 2,2,6,6-tetramethylpiperidin-4-one**

(30) Priority: 01.03.1995 IT MI950389
(71) Applicant: CIBA-GEIGY AG, 4002 Basel (CH); CIBA-GEIGY S.p.A., 21047 Origgio (IT)
(72) Inventor: Carrozza, Primo, 37193 Verona (IT); Ferri, Gianluca, 40011 Anzola Emilia, Bologna (IT)

(57) **Abstract**

A process for methylating 2,2,6,6-tetramethylpiperidin-4-one with formaldehyde and formic acid in an organic solvent, comprising
(A) addition of the formaldehyde to the 2,2,6,6-tetramethylpiperidin-4-one and formation of a 1-hydroxymethyl-2,2,6,6-tetramethylpiperidin-4-one intermediate and
(B) subsequent addition of the formic acid to the 1-hydroxymethyl-2,2,6,6-tetramethylpiperidin-4-one intermediate and formation of 1,2,2,6,6-pentamethylpiperidin-4-one.

## Description

The present invention relates to a process for the methylation of 2,2,6,6-tetramethylpiperidin-4-one.

Several processes for the preparation of 1,2,2,6,6-pentamethylpiperidin-4-one are known. The methylation of 2,2,6,6-tetramethylpiperidin-4-one with methyl iodide is for example described in "Beilsteins Handbuch der Organischen Chemie", 4. edition, volume 21, page 250 and first supplement, page 274 as well as in J. Med. Chem. 6, 381-384 (1963). The reaction of phorone with methylamine to form 1,2,2,6,6-pentamethylpiperidin-4-one is disclosed in "Beilsteins Handbuch der Organischen Chemie", 4. edition, volume 21, second supplement, page 222 and in 3J4. supplement, page 3279.

There is a need for a process wherein the use of potential carcinogenic and toxic reagents such as methyl iodide is avoided.

The methylation of triazine compounds containing 2,2,6,6-tetramethylpiperidin-4-yl groups is for example described in EP-A-319 480. Said methylation is carried out by using a mixture of formaldehyde and formic acid as methylating agent

However, the methylation of 2,2,6,6-tetramethylpiperidin-4-one with a mixture of formaldehyde and formic acid gives a very low yield of methylated product and is, therefore, not acceptable for a production on industrial scale.

The present invention relates to a process for methylating 2,2,6,6-tetramethylpiperidin-4-one with formaldehyde and formic acid in an organic solvent, comprising
(A) addition of the formaldehyde to the 2,2,6,6-tetramethylpiperidin-4-one and formation of a 1-hydroxymethyl-2,2,6,6-tetramethylpiperidin-4-one intermediate and
(B) subsequent addition of the formic acid to the 1-hydroxymethyl-2,2,6,6-tetramethylpiperidin-4-one intermediate and formation of 1,2,2,6,6-pentamethylpiperidin-4-one.

The process is illustrated by the following reaction scheme.

### Scheme:

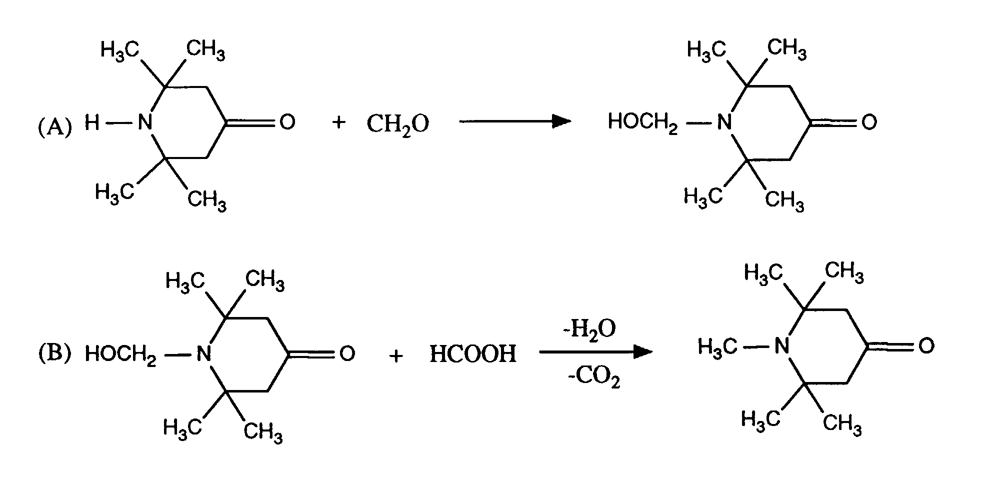

The obtained 1-hydroxymethyl-2,2,6,6-tetramethylpiperidin-4-one intermediate is conveniently not isolated from the reaction mixture since said intermediate is unstable.

It is appropriate to use pure paraformaldehyde and pure formic acid in the process according to the instant invention.

The instant process is preferably carried out in an aromatic hydrocarbon or an alcohol as solvent. An aromatic hydrocarbon solvent is especially preferred. Examples for suitable solvents are benzene, toluene, xylene, trimethylbenzene, chlorobenzene, n-butyl alcohol and its isomers, and t-amyl alcohol. Of particular interest are benzene, toluene and xylene, especially toluene.

According to a further preferred embodiment of the instant invention, the water of reaction is simultaneously separated off by azeotropic distillation in (B).

A preferred ratio of 2,2,6,6-tetramethylpiperidin-4-one : formaldehyde : formic acid is 1:1:1 to 1:3:1.2, particularly 1:1:1 to 1:2:1 or 1:1:1 to 1:1.5:1.

When a stoichiometric ratio of starting materials is used, the ratio of each component can vary within e.g. ±5%.
(A) is preferably carried out at a temperature of 10 to 60°C, particularly 20 to 40°C, and
(B) may be carried out for example at a temperature of 70 to 160°C, particularly 100 to 120°C or 90 to 100°C.

When the reaction has ended, the potential unreacted formic acid and the residual CO₂ are, if desired, neutralized with an aqueous solution of an inorganic base or with a solid inorganic base, preferably NaOH or K₂CO₃. After the aqueous phase (containing unreacted formaldehyde besides formate and carbonate) has been separated off, the organic phase may be distilled to give the desired product.

The 1,2,2,6,6-pentamethylpiperidin-4-one prepared according to the instant process can directly be used as starting material for the preparation of various light stabilizers or, if desired, can be purified by usual methods like crystallization and the like.

The following examples illustrate the present invention more clearly.

Example 1: To a solution of 300 g (1.83 moles) of 2,2,6,6-tetramethylpiperidin-4-one (title 94.5%) in 1500 ml of toluene, 82.2 g (2.74 moles) of paraformaldehyde are added under stirring at room temperature.

Subsequently, the solution is heated to 90°C and 84.2 g (1.83 moles) of formic acid are slowly added, azeotropically distilling off the water of reaction. The solution is then heated to 100°C and maintained at 100°C for 1 hour. After cooling to room temperature, 3.65 g (0.09 moles) of ground sodium hydroxide are added under stirring. The mixture is then stirred for 1 hour and the sodium hydroxide is filtered off. The solution is distilled and 1,2,2,6,6-pentamethylpiperidin-4-one is obtained as a yellow pale oil.

Yield: 275 g = 88.8% of theory

Boiling point: 65-66°C/0.3 mbar (The NMR and GLC-MS analyses conform with 1,2,2,6,6-pentamethylpiperidin-4-one).

Examples 2-4: Following the general procedure disclosed in Example 1 and using the reaction conditions shown in Table 1, the indicated yield of 1,2,2,6,6-pentamethylpiperidin-4-one is obtained.

**Table 1:**

| Example | 2,2,6,6-tetramethyl-piperidin-4-one (moles/title) | CH₂O (moles) | HCOOH (moles) | Solvent | Base | Yield (% of theory) |
|---|---|---|---|---|---|---|
| 2 | 1(100%) | 1.05 | 1.05 | toluene | - | 81% |
| 3 | 1(100%) | 1.50 | 1.00 | toluene | - | 95% |
| 4 | 1(95.3%) | 1.50 | 1.00 | toluene | NaOH | 85% |

Example 5: To a solution of 20 g (0.13 moles) of 2,2,6,6-tetramethylpiperidin-4-one in 100 ml of toluene, 5.8 g (0.195 moles) of paraformaldehyde are added under stirring at room temperature. Subsequently, the solution is heated to 90°C and 6.4 g (0.14 moles) of formic acid are slowly added, azeotropically distilling off the water of reaction. The solution is then heated to 100°C and maintained at 100°C for 1 hour. After cooling to room temperature, 0.66 g (16 moles) of ground sodium hydroxide are added under stirring. The mixture is then stirred for 1 hour and the sodium hydroxide is filtered off. The solution is analyzed by GLC. The conversion to 1,2,2,6,6-pentamethylpiperidin-4-one is 98%. The solution is distilled giving 19.4 g of 1,2,2,6,6-pentamethylpiperidin-4-one (88.2% of theory).

### Methylation of 2,2,6,6-pentamethylpiperidin-4-one with a mixture of formic acid and formaldehyde.

To a solution of 20 g (0.13 moles) of 2,2,6,6-tetramethylpiperidin-4-one in 100 ml of toluene, heated to 90°C, a mixture consisting of 6.4 g (0.14 moles) of formic acid and a solution of 5.8 g (0.195 moles) of paraformaldehyde in 10.5 ml of 2% aqueous NaOH is added slowly, azeotropically distilling off the water of reaction. The solution is then heated to 100°C and maintained at 100°C for 1 hour. After cooling to room temperature, 0.66 g (16 moles) of ground sodium hydroxide are added and the mixture is stirred for a further hour. The residue is filtered off, giving a red-brown solution which has been analyzed by GLC. The conversion to 1,2,2,6,6-pentamethylpiperidin-4-one is 22.7%. The GLC contains 5 further peaks. Accordingly, this process is not acceptable for a production on industrial scale.

## Claims

1. A process for methylating 2,2,6,6-tetramethylpiperidin-4-one with formaldehyde and formic acid in an organic solvent, comprising
(A) addition of the formaldehyde to the 2,2,6,6-tetramethylpiperidin-4-one and formation of a 1-hydroxymethyl-2,2,6,6-tetramethylpiperidin-4-one intermediate and
(B) subsequent addition of the formic acid to the 1-hydroxymethyl-2,2,6,6-tetrarnethylpiperidin-4-one intermediate and formation of 1,2,2,6,6-pentamethylpiperidin-4-one.

2. The process according to claim 1, wherein an aromatic hydrocarbon or an alcohol is used as solvent.

3. The process according to claim 1, wherein benzene, toluene or xylene is used as solvent.

4. The process according to claim 1, wherein water of reaction is simultaneously separated off by azeotropic distillation in (B).

5. The process according to claim 1, wherein a ratio of 2,2,6,6-tetramethylpiperidin-4-one: formaldehyde : formic acid is 1:1:1 to 1:3:1.2.

6. The process according to claim 1, wherein a ratio of 2,2,6,6-tetrarnethylpiperidin-4-one: formaldehyde : formic acid is 1:1:1 to 1:2:1.

7. The process according to claim 1, wherein a ratio of 2,2,6,6-tetramethylpiperidin-4-one : formaldehyde : formic acid is 1:1:1 to 1:1.5:1.

8. The process according to claim 1, wherein a temperature is 10 to 60°C in (A).

9. The process according to claim 1, wherein a temperature is 70 to 160°C in (B).

10. The process according to claim 1, wherein a temperature is 100 to 120°C in (B).
